Europäisches Patentamt

European Patent Office

Office européen des brevets

(11) Veröffentlichungsnummer: **0 373 484**

**A1**

(12) **EUROPÄISCHE PATENTANMELDUNG**

(21) Anmeldenummer: **89122422.2**

(22) Anmeldetag: **05.12.89**

(51) Int. Cl.5: **C07C 53/126, C07C 51/41, //C08K5/09**

(30) Priorität: **14.12.88 DE 3842084**

(43) Veröffentlichungstag der Anmeldung:
**20.06.90 Patentblatt 90/25**

(84) Benannte Vertragsstaaten:
**GR**

(71) Anmelder: **NEYNABER CHEMIE GmbH**
**Am Wedenberg Postfach 11 20**
**D-2854 Loxstedt(DE)**

(72) Erfinder: **Worschech, Kurt, Dr.**
**Alte Strasse 4**
**D-2854 Loxstedt(DE)**
Erfinder: **Fleischer, Erwin**
**Dwarsweg 1**
**D-2850 Bremerhaven-Spaden(DE)**
Erfinder: **Löffelholz, Frido**
**Vieländer Weg 226 F**
**D-2850 Bremerhaven-Surheide(DE)**
Erfinder: **Wedl, Peter**
**Löningstrasse 13**
**D-2850 Bremerhaven(DE)**
Erfinder: **Peters, Artur**
**Im Nordfelde 42**
**D-2854 Loxstedt(DE)**

(54) **Blei/Erdalkali-Komplexe, Verfahren zu ihrer Herstellung und ihre Verwendung als Stabilisatoren für Kunststoffe.**

(57) Blei/Erdalkali-Komplexe der Zusammensetzung

$(PbO)_n\ E(RCOO)_2$

wobei E ein Erdalkalimetall aus der von Calcium und Magnesium gebildeten Gruppe, n eine Zahl von mindestens 1 und die Gruppe RCOO- der Rest einer $C_{12}$-$C_{22}$-Fettsäure ist, stabilisiert durch Partialester mehrwertiger Alkohole, erhältlich durch Umsetzung von PbO und Calcium- bzw. Magnesium-$C_{12}$-$C_{22}$-Fettsäuresalzen in Molverhältnissen von mindestens 1 : 1 in Abwesenheit freier Fettsäuren und in Gegenwart von Partialestern von Polyolen mit 3 bis 15 Kohlenstoffatomen und 2 bis 6 Hydroxylgruppen und Monocarbonsäuren mit 6 bis 32 Kohlenstoffatomen, wobei die Partialester pro Molekül im Durchschnitt mindestens eine freie Hydroxylgruppe enthalten, sind wirksame Stabilisatoren für PVC-Massen.

## Blei/Erdalkali-Komplexe, Verfahren zu ihrer Herstellung und ihre Verwendung als Stabilisatoren für Kunststoffe.

Die Erfindung betrifft Blei/Erdalkali-Komplexe der Zusammensetzung

$(PbO)_n E(RCOO)_2$

wobei E ein Erdalkalimetall aus der von Calcium und Magnesium gebildeten Gruppe, n eine Zahl von mindestens 1 und die Gruppe RCOO- der Rest einer $C_{12}$-$C_{22}$-Fettsäure ist, stabilisiert durch Partialester mehrwertiger Alkohole.

Bleioxid (PbO; Bleiglätte) ist eine intensiv gelbe, feste Verbindung, die in wäßrigen Medien mit Suspensionen von vorgefällten, neutralen Bleisalzen farblose Komplexverbindungen eingeht. Dabei werden in der Regel zweibasische Bleiverbindungen gebildet. Bekannt sind die zweibasischen Komplexe von Bleiphosphit, Bleistearat, Bleiphthalat und Bleifumarat. Diese Komplexe werden insbesondere als Stabilisatoren bei der Herstellung von Formkörpern aller Art aus Hart-PVC eingesetzt. Für diese Zwecke eignen sich weiterhin auch basische Verbindungen des Bleisulfats, insbesondere die dreibasische Variante der Formel

$3PbO \times PbSO_4$.

Derartige Stabilisatorsysteme werden häufig durch Calciumseifen, insbesondere Calciumstearat, komplettiert, die allerdings auch Gleitmittel- bzw. Schmiereigenschaften aufweisen, so daß sie häufig mit Paraffinen, anderen Kohlenwasserstoffwachsen, wie Polyethylenderivaten, freien Fettsäuren und gelegentlich auch Fettsäureestern eingesetzt werden.

Aus der DE-A 34 44 259 ist bekannt, daß sich basische Bleiseifen der Zusammensetzung

$2PbO.Pb(Fettsäurerest)_2$

nicht nur in wäßriger Lösung, sondern auch in einer Schmelze herstellen lassen, wenn die Zugabe von Bleioxid zum geschmolzenen Bleistearat in Gegenwart bestimmter polarer Substanzen, insbesondere Partialestern mehrwertiger Alkohole, z.B. solchen des Glycerins und/oder des Pentaerythrits, erfolgt.

Die Erfindung beruht auf der Erkenntnis, daß sich basische Bleiverbindungen auch dann bilden, wenn man Erdalkaliseifen, z.B. Calcium- oder Magnesiumstearate, als Substrate für die Herstellung einer basischen PbO-Mischseife verwendet. Voraussetzung ist auch hier, daß der Aufbau zur basischen Seife in Gegenwart von polaren Schmelzpartnern, insbesondere Partialestern, erfolgt. Die hierbei ablaufende chemische Reaktion läßt sich sehr schön mit den folgenden Versuchen demonstrieren, bei denen die Indikatorwirkung des intensiv gelben Bleioxids ausgenutzt wird:

In ein Schmelzgemisch von 50 g Pentaerythrit-distearat und 50 g Calciumstearat wurden bei 150°C unter Rühren ca. 20 g Bleioxid eingetragen. Die deutlich gelbe Farbe des Bleioxids war nach ca. 15 min praktisch verschwunden; gleichzeitig stieg die Viskosität der Suspension stark an. Nach Zusatz von 25 g Paraffin und Temperaturerhöhung auf 160°C wurde die Schmelze dünncremig, d.h. gießfähig. Die Schmelzfarbe war relativ hell. In dieser Form konnte die Schmelze konfektioniert, z.B. gesprüht oder pastelliert, werden. Bei einer Wiederholung des Versuches in modifizierter Form ohne Ca-stearat wurde Bleioxid in geschmolzenem Pentaerythrit-distearat suspendiert. Auch nach 30 min bei 150°C unter Rühren hatte sich die gelbe Schmelzfarbe nicht verändert. Nach Eintragen von Calciumstearat (50%, berechnet auf Bleioxid) erfolgte eine rasche Entfärbung der Suspension; es lag eine hellcremige Schmelze vor.

Insbesondere bei der Versuchswiederholung wird deutlich, daß hier pro Molekül Calciumstearat zwei Moleküle Bleioxid chemisch gebunden werden können. Für das Zustandekommen und vermutlich auch für die Stabilisierung des erhaltenen Blei/Calcium-Komplexes ist die Gegenwart eines polaren schmelzbaren Lösemittels, z.B. eines Partialesters, unbedingt erforderlich.

Demgemäß ist die Erfindung auf Blei/Erdalkali-Komplexe der eingangs genannten Zusammensetzung gerichtet, die durch Umsetzung von Bleioxid (PbO) und Calcium- bzw. Magnesium-$C_{12}$-$C_{22}$-Fettsäuresalzen in Molverhältnissen von mindestens 1 : 1 in Abwesenheit freier Fettsäuren und in Gegenwart von Partialestern von Polyolen mit 3 bis 5 Kohlenstoffatomen und 2 bis 6 Hydroxylgruppen und Monocarbonsäuren mit 6 bis 32 Kohlenstoffatomen, wobei die Partialester pro Molekül im Durchschnitt mindestens eine freie Hydroxylgruppe enthalten, erhältlich sind.

Die Blei/Erdalkali-Komplexe der Erfindung weisen den Vorteil auf, daß sie nicht mehr das als stark schmierend bekannte neutrale Bleistearat enthalten, so daß "Überschmierungen" stabilisierter Massen auf Basis von PVC vermieden werden können. Die Blei/Erdalkali-Komplexe der Erfindung enthalten vielmehr Bleioxid in reaktiver Form, ohne daß zu ihrer Herstellung gefällte Produkte wie 2-basisches Bleistearat (51% Pb) oder 3-basisches Bleisulfat als Trägersubstanzen benötigt werden. Auf den Primärstabilisator Bleioxid kann vielmehr direkt im Schmelzverfahren auch ein preiswertes, ohnehin gebräuchliches Substrat wie Calcium- und/oder Magnesiumstearat aufgebracht werden. Gegenüber dem Auftrag auf neutrales Bleistearat hat dies insoweit einen erheblichen Vorteil, als zunächst ein isoliertes Stabilisierungssystem hergestellt werden kann, das anschließend rheologisch modifizierbar ist. Diese Art der Rezepturherstellung kann nach dem Stand der Technik nur mit teueren Einzelkomponenten erfolgen; dabei werden zum Teil inerte Verbindungen wie Bleisulfat eingebracht, die nur als Pigmente bzw. Träger für Bleioxid wirken.

Die Anzahl der PbO-Moleküle, die sich auf ein Molekül Calcium-oder Magnesium-Fettsäuresalz aufbringen läßt, ist nicht kritisch; sie beträgt bevorzugt bis zu 30, kann jedoch auch über 30 liegen. Dabei ist allenfalls zu berücksichtigen, daß derartige Komplexe mit hohem PbO-Anteil (n>8) einen für Stabilisator/Gleitmittelkombinationen zu geringen Calciumgehalt aufweisen können, der jedoch durch geeignete Maßnahmen, z.B. einen Zusatz entsprechender Magnesium-und/oder Calcium-Fettsäuresalze zu der Stabilisator/Gleitmittelkombination ausgeglichen werden kann.

Typische Beispiele für die Gruppe RCOO- der eingangs genannten Formel bildenden $C_{12}$-$C_{22}$-Fettsäuren, die natürlicher und/oder synthetischer Herkunft sein können, sind Laurin-, Myristin-, Palmitin-, Stearin-, Behen-, Montan-, Öl-, Petroselin-, Palmitolein- und Erucasäure; außer den vorgenannten geradkettigen Fettsäuren, die bevorzugt sind, können auch verzweigte Fettsäuren der entsprechenden Kohlenstoffzahl eingesetzt werden. Bevorzugt sind geradkettige Fettsäuren der angegebenen Kohlenstoffzahl, die, wie in der Fettchemie üblich, in Form ihrer technischen Gemischen eingesetzt werden und aus natürlichen Rohstoffen tierischer oder pfanzlicher Herkunft zugänglich sind, z.B. aus Cocos-, Palmkern-, Sonnenblumen-, Raps-, Rübsen- und Korianderöl sowie aus Rinder- und Schweinetalg. Besonders bevorzugt sind hier die Calcium-und/oder Magnesium-Fettsäuresalze, die von geradkettigen, gesättigten Fettsäuren aus der von Laurin-, Myristin-, Palmitin-, Stearin- und Behensäure gebildeten Gruppe abgeleitet sind. Besonders vorteilhaft sind für die Bildung der Blei/Erdalkali-Komplexe der Erfindung Magnesium-und/oder Calcium-Stearate.

Wie bereits ausgeführt wurde, erfordern die Blei/Erdalkali-Komplexe der Erfindung, daß ihre Herstellung in der Abwesenheit freier Fettsäuren erfolgt, um die Bildung von Blei-Fettsäuresalzen zu verhindern. Für ihre Stabilisierung ist weiterhin die Anwesenheit von Partialestern von Polyolen mit 3 bis 15 Kohlenstoffatomen und 2 bis 6 Hydroxylgruppen und Monocarbonsäuren mit 6 bis 32 Kohlenstoffatomen erforderlich, wobei die Partialester pro Molekül im Durchschnitt mindestens eine freie Hydroxylgruppe enthalten. Als Polyole zur Bildung der genannten Partialester kommen insbesondere Glycerin, Trimethylolpropan, Pentaerythrit, Diglycerin, Triglycerin, Di-trimethylolpropan, ni-pentaerythrit und Tetraglycerin in Frage. Besonders bevorzugt sind hier Pentaerythrit, Glycerin und Diglycerin. Die Fettsäurekomponente der oben genannten Partialester kann von den oben genannten Fettsäuren natürlicher und/oder synthetischer, insbesondere natürlicher Herkunft einschließlich Capron-, Capryl-, Caprin-, Linolen-und 12-Hydroxystearinsäure gebildet sein; bevorzugt sind Partialester in Form von Umsetzungsprodukten des Pentaerythrits, des Glycerins oder Diglycerins mit Monocarbonsäuren aus der von Laurin-, Myristin-, Palmitin-, Stearin-, Behen-, Öl-, Linolen-, 12-Hydroxystearin- und Montansäure gebildeten Gruppe mit OH-Zahlen von 140 bis 580, insbesondere 170 bis 450, und Säurezahlen von weniger als 15, insbesondere weniger als 8. Besonders bevorzugte Partialester sind die Distearate des Pentaerythrits, des Glycerins und des Diglycerins.

Gemäß einer vorteilhaften Ausführungsform der Erfindung sind die Blei/Erdalkali-Komplexe durch die Zusammensetzung $(PbO)_n E(Stearat)_2$, gekennzeichnet, in der n eine Zahl im Bereich von 1 bis 8 und E wie oben definiert ist; diese Komplexe sind stabilisiert durch die Partialester mehrwertiger Alkohole und erhältlich durch die Umsetzung von 1 Mol Calcium- bzw. Magnesiumdistearat mit 1 bis 8 Mol in dem Partialester suspendiertem PbO bei Temperaturen oberhalb des Schmelzpunktes der Partialester.

Die Erfindung betrifft weiterhin die Herstellung der Blei/Erdalkali-Komplexe der Erfindung durch Verfahren mit den bereits genannten Merkmalen. Dabei werden die Konzentrationsbereiche der eingesetzten Reaktanten (PbO, Magnesium- und/oder Calcium Fettsäuresalze, bzw. deren Vorläufer, insbesondere MgO, $Mg(OH)_2$, CaO und/oder $Ca(OH)_2$ sowie stöchiometrische Mengen an Fettsäuren, und Partialester) so gewählt, daß nach Beendigung der Reaktion der Pb-Gehalt im Reaktionsmedium zwischen 5 und 30, insbesondere zwischen 8 und 25 Gew.-%, bezogen auf das Gesamtgewicht des Reaktionsgemisches, beträgt. Allgemein kann die Herstellung so erfolgen, daß man in den vorgelegten Partialestern zunächst aus den entsprechenden Erdalkalioxiden bzw. -hydroxiden und Fettsäuren die Erdalkalisalze erzeugt und anschließend PbO in der Stöchiometrie der erfindungsgemäß herzustellenden Blei/Erdalkalikomplexe entsprechenden Mengen einträgt; selbstverständlich kann man jedoch auch die Erdalkali-Fettsäuresalze getrennt herstellen und mit PbO in Gegenwart der Partialester umsetzen. Im übrigen können die Blei/Erdalkalikomplexe der Erfindung auch in Gegenwart eines Überschusses an Erdalkalifettsäuresalzen hergestellt werden; man erhält dann die Komplexe der Erfindung im Gemisch mit nicht umgesetzten Erdalkalifettsäuresalzen, so daß sich rechnerisch eine Zusammensetzung z.B. der Formel $(PbO)_{\geq 0,2}E(RCOO)_2$ für das Reaktionsgemisch ergibt.

Die Erfindung betrifft weiterhin Stabilisatoren für Kunststoffe auf Basis von Polyvinylchlorid oder Copolymeren desselben, die die mit Partialestern mehrwertiger Alkohole stabilisierten Blei/Erdalkali-Komplexe der Erfindung enthalten.

Die Erfindung wird im folgenden anhand von bevorzugten Beispielen näher erläutert.

Beispiel 1.

$(PbO)_{3,9}Ca(Stearat)_2$

In einem offenen Glasgefäß mit Rührer und Thermometer wurden 200 g Pentaerythrit-distearat und 22,4 g (0,083 Mol) einer technischen Stearinsäure aufgeschmolzen und auf 150°C erhitzt. Unter Rühren wurden anschließend im Verlauf von 2 min 3,1 g (0,042 Mol) Calciumhydroxid in die Schmelze eingetragen, wobei die Reaktionstemperatur auf 150°C gehalten wurde. Nach 5-minütigem Nachrühren resultierte eine fast blanke, gelbliche Schmelze. Es wurden nun im Verlauf von 30 min 36 g (0,16 Mol) Bleiglätte jeweils in kleinen Portionen eingerührt. Nach der letzten Zugabe wurde 10 min nachgerührt. Die Bleiglätte hatte sich restlos umgesetzt. Die Reaktionsmischung lag ohne Bodenkörper als sämige, weißlich-braunstichige Schmelze vor, die beim Abkühlen erstarrte.
Kenndaten:
Smp. 104°C (Kofler)
Pb-Gehalt 12,9%
PbO-Gehalt 13,9%
Ca-Gehalt 0,64%
Asche (1000°C) 14,6% (ber. 14,8%)
Säurezahl 0,35

Beispiel 2.

$(PbO)_{6,3}Ca(Stearat)_2$

Analog Beispiel 1 wurden 200 g Pentaerythrit-distearat und 14,9 g (0,055 Mol) technische Stearinsäure auf 150°C erhitzt und mit 2,1 g (0,028 Mol) Calciumhydroxid versetzt. Nach der Umsetzung wurden 39 g (0,175 Mol) Bleiglätte in 35 min portionsweise eingetragen. Die Schmelze wurde bei 150°C 10 min nachgerührt. Die Reaktionsmischung lag als sämige, weißlichbraunstichige Schmelze ohne Bodenkörper vor, die beim Abkühlen erstarrte.
Kenndaten:
Smp.(Kofler) 100°C
Ca-Gehalt 0,43%
Pb-Gehalt 14,2%

Asche (1000°C) 15,6% (ber. 15,9%)
Säurezahl 0,5

Beispiel 3.

$(PbO)_{1,8}Ca(Stearat)_2$.

Analog Beispiel 1 wurden 140 g Diglycerindistearat und 74,8 g (0,277 Mol) technische Stearinsäure auf 150°C erhitzt und 10,2 g (0,138 Mol) Calciumhydroxid in 5 min eingerührt. Nach der Umsetzung des Calciumhydroxids wurden 55 g (0,246 Mol) Bleiglätte in 10 min eingetragen. Die Schmelze wurde bei 150°C weitere 10 min nachgerührt, bis die Bleiglätte umgesetzt war. Es lag eine sämige, bräunliche Schmelze vor, die beim Abkühlen erstarrte [Smp. 94°C (Kofler)].

Beispiel 4.

$(PbO)_{27}Ca(Stearat)_2$.

Analog zu der in Beispiel 1 beschriebenen Weise wurden 81,6 Gew.-Teile Pentaerythrit-distearat, 1,6 Gew.-Teile Calciumstearat und 16,7 Gew.-Teile Bleiglätte umgesetzt. Trotz des hohen molaren PbO/Ca-Distearat-Verhältnisses von 27 : 1 wurde unter Entfärbung des PbO eine Schmelze erhalten, die beim Abkühlen zu einer beigefarbenen, etwas hartwachsigen Masse erstarrte.
Kenndaten:
Smp.(Kofler) 100°C
Ca-Gehalt 0,11%
Pb-Gehalt 15,5%
Asche(1000°C) 16,5% (ber. 16,8%)
Säurezahl 0,4

Beispiel 5.

$(PbO)_{2,5}Mg(Stearat)_2$.

In einem offenen Glasgefäß mit Rührer und Thermometer wurden 160 g Pentaerythrit-distearat und 19,2 g (0,07 Mol) technische Stearinsäure auf 150°C erhitzt. Unter Rühren wurden 1,4 g (0,035 Mol) Magnesiumoxid innerhalb von 2 min in die Schmelze eingetragen und 30 min nachgerührt. Es resultierte eine fast blanke, gelbe Schmelze. Im Verlauf von 5 min wurden anschließend 20 g (0,09 Mol) Bleiglätte portionsweise eingerührt und bis zur Entfärbung der Schmelze 20 min nachgerührt. Die Reaktionsmischung lag als getrübte, weißlich-braustichige, schwach viskose Schmelze ohne Bodensatz vor, die beim Abkühlen erstarrte.
Kenndaten:
Fließ-Schmelzpunkt 91,0 °C
Mg-Gehalt 0,43%
Pb-Gehalt 9,3%
Asche (1000 °C) 10,7% (ber. 10,7%)
Säurezahl 2,3

Beispiel 6.

$(PbO)_2Mg(Stearat)_2$.

Analog Beispiel 5 wurden 128,2 g Pentaerythrit-distearat und 38,4 g (0,14 Mol) technische Stearinsäure auf 150 °C erhitzt und 2,8 g (0,07 Mol) Magnesiumoxid in 3 min eingerührt. Nach 10-minütigem Rühren lag eine fast blanke, gelbe Schmelze vor. Im Verlauf von 10 min wurden unter Rühren 31,8 g (0,14 Mol) Bleiglätte eingetragen und 15 min bis zur Entfärbung der Schmelze nachgerührt. Man erhielt eine weißlich-braunstichige, sehr schwach viskose Schmelze, die beim Abkühlen erstarrte.
Kenndaten:
Fließ-Schmelzpunkt 92,5 °C
Mg-Gehalt 0,85%
Pb-Gehalt 14,8%
Asche (1000 °C) 17,4% (ber. 17,3%)
Säurezahl 2,4

Beispiel 7.

$(PbO)_2Mg(Stearat)_2$.

Analog Beispiel 5 wurden 144,4 g Pentaerythrit-distearat und 115,3 g (0,43 Mol) technisches Stearinsäure auf 150 °C erhitzt und mit 8,6 g (0,215 Mol) Magnesiumoxid versetzt. Nach der Umsetzung des Magnesiumoxids wurden 95,6 g (0,43 Mol) Bleiglätte in 15 min eingetragen und 1 h bis zur Entfärbung der Schmelze gerührt. Man erhielt eine schwach getrübte, bräunlich-gelbe, mäßig viskose Schmelze ohne Bodenkörper, die beim Abkühlen erstarrte (Smp. 96 °C).

Beispiel 8.

$(PbO)_2Mg(Stearat)_2$.

Analog Beispiel 5 wurden 56,4 g Pentaerythrit-distearat und 76,9 g (0,28 Mol) technisches Stearinsäure auf 150 °C erhitzt und 5,7 g (0,14 Mol) Magnesiumoxid eingerührt. Nach der Umsetzung lag eine fast blanke, gelbe, mäßig viskose Schmelze vor. Anschließend wurden in 10 min 63,6 g (0,28 Mol) Bleiglätte eingetragen und 15 min nachgerührt. Es lag eine dünnsämige, weißlich-braunstichige Schmelze vor, die beim Abkühlen erstarrte.
Kenndaten:
Fließ-Schmelzpunkt 112,0 °C
Mg-Gehalt 1,7%
Pb-Gehalt 29,5%
Asche (1000 °C) 34,7% (ber. 34,6%)
Säurezahl 2,2

Vergleichsbeispiele.

Mit den Blei/Erdalkali-Komplexen der Erfindung stabilisierte PVC-Formmassen wurden zur Ermittlung ihrer statischen Stabilität auf einem Laborwalzwerk der Abmessung 450x220mm (Fa. Berstorff) bei einer Walzentemperatur von 170 °C und einer Walzendrehzahl von 12,5 U/min im Gleichlauf im Verlauf von 5 min zu Prüffellen verarbeitet. Die ca. 0,5 mm dicken Felle wurden zu quadratischen Probestücken mit 10 mm Kantenlänge zerschnitten und anschließend in einem Trockenschrank mit 6 rotierenden Horden (Heraeus FT 420R) einer Temperatur von 180 °C ausgesetzt. Im Abstand von 15 min wurden Proben entnommen; die Farbänderungen wurden bestimmt. Es wird für jede Formmasse im folgenden zunächst unter "Anfangsstabilität" diejenige Zeit angegeben, nach der die Proben eine deutliche Verfärbung zeigten. Unter "Stabilitätsabbruch" ist diejenige Zeit angegeben, nach der der Test wegen zu starker Verfärbung abgebrochen wurde.

Es wurde jeweils die stabilisierende Wirkung eines Blei/Erdalkali-Komplexes (a) der Erfindung in PVC-Massen der unter "Grundansatz" angegebenen Zusammensetzung mit derjenigen eines Pb/Ca-Stabilisatorsystems (b) mit entsprechenden Blei-, Calcium- bzw. Magnesium-, Stearat- und Partialestergehalten gemäß Stand der Technik verglichen.

Weiterhin wurde das Plastizierverhalten der in den Vergleichsbeispielen hergestellten Formmassen mit Hilfe eines Plastographen (Plasticorder PL 151; Fa. Brabender; siehe P. Klenk, "Der Plastverarbeiter", 21. Jahrgang, 1970/7, S. 642 bis 644) geprüft. Die Kammertemperatur betrug 165 °C, die Drehzahl 30 U/min. Die Untersuchungen wurden mit jeweils 34 g stabilisierter PVC-Masse ausgeführt.

Vergleichsbeispiel 1.

Grundansatz: 100 Gew.-Teile Suspensions-PVC, K-Wert 60

    a) 2,5 Gew.-Teile $(PbO)_{3,9}Ca(Stearat)_2$ - (Beispiel 1)

0,1 Gew.-Teile Paraffinwachs

    b) 0,35 Gew.-Teile Bleisulfat

0,25 Gew.-Teile Ca-Stearat

2,0 Gew.-Teile Pentaerythrit-distearat

0,1 Gew.-Teile Paraffinwachs Anfangsfarbe:

    a) ganz leicht gelblich

    b) leicht gelblich Anfangsstabilität:

    a) 20 min leicht gelblich

40 min deutlich gelblich

    b) 5 min deutlich gelblich Stabilitätsabbruch:

    a) 70 min

    b) 50 min Plastizierzeit:

    a) 13 min

    b) 7,8 min

Vergleichsbeispiel 2.

Grundansatz wie bei Vergleichsbeispiel 1

    a) 2,55 Gew.-Teile $(PbO)_{6,3}Ca(Stearat)_2$ - (Beispiel 2)

0,1 Gew.-Teile Paraffinwachs

    b) 0,4 Gew.-Teile Pb-Sulfat

0,15 Gew.-Teile Ca-Stearat

2,0 Gew.-Teile Pentaerythrit-distearat

0,1 Gew.-Teile Paraffinwachs

Anfangsfarbe:

    a) ganz leicht gelblich

    b) leicht gelblich Anfangsstabilität:

    a) 15 min leicht gelblich

30 min deutlich gelblich

    b) 5 min deutlich gelblich Stabilitätsabbruch:

    a) 70-80 min

    b) 50 min Plastizierzeit:

    a) 14,2 min

    b) 7,4 min

Vergleichsbeispiel 3.

Grundansatz: 100 Gew.-Teile Hart-PVC wie bei Vergleichsbeispiel 1, 0,1 Gew.-Teile Paraffinwachs, 0,5 Gew.-Teile Bleisulfat.

    a) 2,0 Gew.-Teile $(PbO)_{2,5}Mg(Stearat)_2$ - (Beispiel 5)

    b) 0,2 Gew.-Teile PbO

0,2 Gew.-Teile Mg-Stearat

1,6 Gew.-Teile Pentaerythrit-distearat Anfangsfarbe:

    a) leicht gelblich

    b) leicht gelblich Anfangsstabilität:

    a) 30 min deutlich gelblich

    b) 15 min deutlich gelblich Stabilitätsabbruch:

    a) 75 min

    b) 50 min Plastizierzeit:

    a) 3,2 min

    b) 2,1 min

Vergleichsbeispiel 4.

Grundansatz wie im Vergleichsbeispiel 3.

    a) 2,0 Gew.-Teile $(PbO)_2Mg(Stearat)_2$ - (Beispiel 6)

    b) 0,32 Gew.-Teile PbO

0,4 Gew.-Teile Mg-Stearat

1,28 Gew.-Teile Pentaerythrit-distearat Anfangsfarbe:

    a) leicht gelblich

    b) leicht gelblich Anfangsstabilität:

    a) 45 min deutlich gelblich

    b) 30 min deutlich gelblich Stabilitätsabbruch:

    a) 105 min

    b) 75 min Plastizierzeit:

    a) 4,5 min

    b) 3,1 min

Vergleichsbeispiel 5.

Grundansatz wie im Vergleichsbeispiel 3.

    a) 2,0 Gew.-Teile $(PbO)_2Mg(Stearat)_2$ - (Beispiel 7)

    b) 0,2 Gew.-Teile PbO

0,2 Gew.-Teile Mg-Stearat

1,6 Gew.-Teile Pentaerythrit-distearat Anfangsfarbe:

    a) ganz leicht gelblich

    b) leicht gelblich Anfangsstabilität:

    a) 15 min leicht gelblich

45 min deutlich gelblich

    b) 15 min deutlich gelblich Stabilitätsabbruch:

    a) 90 min

    b) 50 min

Die Wiederholung dieses Vergleichsbeispiels mit $(PbO)_2Mg(Stearat)_2$ gemäß Beispiel 8 ergab die gleichen Stabilitätswerte wie die entsprechende Verbindung des Beispiels 7; die Plastizierzeiten waren für a) 5,0 min und für b) 1,7 min.

Es zeigte sich, daß die Blei/Erdalkali-Komplexe der Erfindung deutlich bessere Stabilitätswerte als diejenigen der Vergleichssysteme ergaben. Weiterhin ergaben sich für die mit den Blei/Erdalkali-Komplexen stabilisierten PVC-Massen deutlich verlängerte Plastizierzeiten.

**Ansprüche**

1. Blei/Erdalkali-Komplexe der Zusammensetzung

$(PbO)_n E(RCOO)_2$

wobei E ein Erdalkalimetall aus der von Calcium und Magnesium gebildeten Gruppe, n eine Zahl von mindestens 1 und die Gruppe RCOO- der Rest

einer $C_{12}$-$C_{22}$-Fettsäure ist, stabilisiert durch Partialester mehrwertiger Alkohole, erhältlich durch Umsetzung von PbO und Calcium- bzw. Magnesium-$C_{12}$-$C_{22}$-Fettsäuresalzen in Molverhältnissen von mindestens 1 : 1 in Abwesenheit freier Fettsäuren und in Gegenwart von Partialestern von Polyolen mit 3 bis 15 Kohlenstoffatomen und 2 bis 6 Hydroxylgruppen und Monocarbonsäuren mit 6 bis 32 Kohlenstoffatomen, wobei die Partialester pro Molekül im Durchschnitt mindestens eine freie Hydroxylgruppe enthalten.

2. Blei/Erdalkali-Komplexe nach Anspruch 1, dadurch gekennzeichnet, daß die Fettsäurereste der Calcium- bzw. Magnesium-$C_{12}$-$C_{22}$-Fettsäuresalze aus der von Laurin-, Myristin-, Palmitin-, Stearin- und Behensäureresten gebildeten Gruppe ausgewählt sind.

3. Blei/Erdalkali-Komplexe nach Anspruch 1 oder 2, dadurch gekennzeichnet, daß die Polyolkomponente der Partialester aus der von Glycerin, Diglycerin, Triglycerin, Tetraglycerin, Trimethylolpropan, Ditrimethylolpropan, Pentaerythrit und Dipentaerythrit gebildeten Gruppe ausgewählt ist.

4. Blei/Erdalkali-Komplexe nach mindestens einem der Ansprüche 1 bis 3, dadurch gekennzeichnet, daß die Polyolkomponente der Partialester Pentaerythrit ist.

5. Blei/Erdalkali-Komplexe nach mindestens einem der Ansprüche 1 bis 3, dadurch gekennzeichnet, daß die Polyolkomponente der Partialester Glycerin oder Diglycerin ist.

6. Blei/Erdalkali-Komplexe nach mindestens einem der Ansprüche 1 bis 5, dadurch gekennzeichnet, daß die Partialester Umsetzungsprodukte des Pentaerythrits, Glycerins oder Diglycerins mit Monocarbonsäuren aus der von Laurin-, Myristin-, Palmitin-, Stearin-, Behen-, Öl-, Linolen-, 12-Hydroxystearin- und Montansäure gebildeten Gruppe mit OH-Zahlen von 140 bis 580, insbesondere 170 bis 450, und Säurezahlen von weniger als 15, insbesondere weniger als 8, sind.

7. Blei/Erdalkali-Komplexe nach mindestens einem der Ansprüche 1 bis 6, dadurch gekennzeichnet, daß der Partialester Pentaerythritdistearat ist.

8. Blei/Erdalkali-Komplexe nach mindestens einem der Ansprüche 1 bis 6, dadurch gekennzeichnet, daß der Partialester Glycerindistearat oder Diglycerindistearat ist.

9. Blei/Erdalkali-Komplexe nach mindestens einem der Ansprüche 1 bis 8, gekennzeichnet durch die Zusammensetzung

$(PbO)_n$ $E(Stearat)_2$,

in der n eine Zahl im Bereich von 1 bis 8 und E wie oben definiert ist, stabilisiert durch die Partialester mehrwertiger Alkohole, erhältlich durch Umsetzung von 1 Mol Calcium- bzw. Magnesiumdistearat mit 1 bis 8 Mol in dem Partialester suspendiertem PbO bei Temperaturen oberhalb des Schmelzpunktes der Partialester.

10. Verfahren zur Herstellung von Blei/Erdalkali-Komplexen der Zusammensetzung $(PbO)_n$ $E(RCOO)_2$ wobei E ein Erdalkalimetall aus der von Calcium und Magnesium gebildeten Gruppe, n eine Zahl von mindestens 1 und die Gruppe RCOO- der Rest einer $C_{12}$-$C_{22}$-Fettsäure ist, stabilisiert durch Partialester mehrwertiger Alkohole, dadurch gekennzeichnet, daß man PbO und Calcium- bzw. Magnesium-$C_{12}$-$C_{22}$-Fettsäuresalze in Molverhältnissen von mindestens 1 : 1 in Abwesenheit freier Fettsäuren und in Gegenwart von Partialestern von Polyolen mit 3 bis 15 Kohlenstoffatomen und 2 bis 6 Hydroxylgruppen und Monocarbonsäuren mit 6 bis 32 Kohlenstoffatomen, wobei die Partialester pro Molekül im Durchschnitt mindestens eine freie Hydroxylgruppe enthalten, umsetzt.

11. Verfahren nach Anspruch 10, dadurch gekennzeichnet, daß man Calcium- bzw. Magnesium-$C_{12}$-$C_{22}$-Fettsäuresalze verwendet, deren Fettsäurereste aus der von Laurin-, Myristin-, Palmitin-, Stearin- und Behensäureresten gebildeten Gruppe ausgewählt sind.

12. Verfahren nach Anspruch 10 oder 11, dadurch gekennzeichnet, daß man Partialester verwendet, deren Polyolkomponente aus der von Glycerin, Diglycerin, Triglycerin, Tetraglycerin, Trimethylolpropan, Ditrimethylolpropan, Pentaerythrit und Dipentaerythrit gebildeten Gruppe ausgewählt sind.

13. Verfahren nach mindestens einem der Ansprüche 10 bis 12, dadurch gekennzeichnet, daß man Partialester verwendet, deren Polyolkomponente von Pentaerythrit gebildet ist.

14. Verfahren nach mindestens einem der Ansprüche 10 bis 12, dadurch gekennzeichnet, daß man Partialester verwendet, deren Polyolkomponente von Glycerin oder Diglycerin gebildet ist.

15. Verfahren nach mindestens einem der Ansprüche 10 bis 14, dadurch gekennzeichnet, daß man Partialester verwendet, die Umsetzungsprodukte des Pentaerythrits, Glycerins oder Diglycerins mit Monocarbonsäuren aus der von Laurin-, Myristin-, Palmitin-, Stearin-, Behen-, Öl-, Linolen-, 12-Hydroxystearin- und Montansäure gebildeten Gruppe mit OH-Zahlen von 140 bis 580, insbesondere 170 bis 450, und Säurezahlen von weniger als 15, insbesondere weniger als 8, sind.

16. Verfahren nach mindestens einem der Ansprüche 10 bis 15, dadurch gekennzeichnet, daß man als Partialester Pentaerythritdistearat verwendet.

17. Verfahren nach mindestens einem der Ansprüche 10 bis 15, dadurch gekennzeichnet, daß man als Partialester Glycerindistearat oder Diglycerindistearat verwendet.

18. Verfahren nach mindestens einem der Ansprüche 10 bis 17, zur Herstellung von Blei/Erdalkali-Komplexen der Zusammensetzung

$$(PbO)_n E(Stearat)_2,$$

in der n eine Zahl im Bereich von 1 bis 8 und E wie oben definiert ist, stabilisiert durch die Partialester mehrwertiger Alkohole, dadurch gekennzeichnet, daß man 1 Mol Calcium- bzw. Magnesiumdistearat mit 1 bis 8 Mol in dem Partialester suspendiertem PbO bei Temperaturen oberhalb der Schmelzpunkte der Partialester umsetzt.

19. Stabilisator für Kunststoffe auf Basis von Polyvinylchlorid oder Copolymeren desselben, enthaltend mit Partialestern mehrwertiger Alkohole stabilisierte Blei/Erdalkali-Komplexe nach mindestens einem der Ansprüche 1 bis 9.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| E | EP-A-0 330 097  (NEYNABER) <br> * Ansprüche 1-3; Spalte 1, Zeilen 20-24 * <br> --- | 1,10,19 | C 07 C  53/126 <br> C 07 C  51/41 // <br> C 08 K  5/09 |
| D,Y | EP-A-0 184 128  (NEYNABER) <br> * Ansprüche 1-12; Seite 5, Zeilen 3-5 - Seite 6, Zeile 24; Seite 9, Zeilen 10-26 * <br> --- | 1-19 | |
| Y | EP-A-0 184 129  (NEYNABER) <br> * Ansprüche 1-10; Seite 8, Zeilen 11-26 * <br> --- | 1-19 | |
| A | US-A-3 072 693  (SZCZEPANEK et al.) <br> * Anspruch 1; Spalte 9, Zeilen 25-32 * <br> ----- | 1,19 | |

| | | | RECHERCHIERTE SACHGEBIETE (Int. Cl.5) |
|---|---|---|---|
| | | | C 07 C  53/00 <br> C 07 C  51/00 |

Der vorliegende Recherchenbericht wurde für alle Patentansprüche erstellt

| Recherchenort | Abschlußdatum der Recherche | Prüfer |
|---|---|---|
| DEN HAAG | 22-02-1990 | KLAG M.J. |